# EUROPÄISCHE PATENTSCHRIFT

(11) **EP 1 490 052 B1**
(45) Veröffentlichungstag und Bekanntmachung des Hinweises auf die Patenterteilung: **30.05.2007**
(21) Anmeldenummer: 03789421.9
(22) Anmeldetag: 24.12.2003
(51) Int. Cl.: A61K 31/381, A61K 9/70

(54) **VORRICHTUNG ZUR TRANSDERMALEN VERABREICHUNG VON ROTIGOTIN-BASE**
DEVICE FOR THE TRANSDERMAL ADMINISTRATION OF A ROTIGOTINE BASE
DISPOSITIF POUR L'ADMINISTRATION TRANSDERMIQUE DE BASE DE ROTIGOTINE

(30) Priorität: 30.12.2002 DE 10261696
(43) Veröffentlichungstag der Anmeldung: 29.12.2004
(73) Patentinhaber: SCHWARZ PHARMA AG, 40789 Monheim (DE)
(72) Erfinder: BREITENBACH, Armin, 51371 Leverkusen (DE)
(86) Internationale Anmeldenummer: PCT/EP2003/014902
(87) Internationale Veröffentlichungsnummer: WO 2004/058247

(56) Entgegenhaltungen:
- EP-A- 1 256 340
- US-A- 5 658 975

## Beschreibung

Die vorliegende Erfindung betrifft eine zur transdermalen Verabreichung von Rotigotin [(-)-5,6,7,8-Tetrahydro-6-[propyl[2-(2-thienyl)ethyl]amino]-1-naphtol] geeignete Matrix, die frei von Löslichkeitsvermittlern und Dispersionsmitteln ist und die im wenigstens ein Matrixpolymer und Rotigotin-Base in einer Konzentration oberhalb der Löslichkeitsgrenze des Matrixpolymers für Rotigotin umfasst, wobei der nicht im Matrixpolymer gelöste Anteil des Rotigotins als amorphe Partikel mit einem mittleren Durchmesser von maximal 30 µm im Matrixpolymer dispergiert ist.

Ferner betrifft die Erfindung eine flächenförmige Vorrichtung zur transdermalen Verabreichung von Rotigotin, die die oben beschriebene, mit Rotigotin übersättigte, vorzugsweise silikonbasierte Matrix und eine für den Wirkstoff undurchlässige Rückschicht enthält.

Aus dem Stand der Technik sind verschiedene silikonbasierte transdermale Systeme zur Verabreichung von Rotigotin bekannt.

WO 94-07468 offenbart ein transdermales System, das ein Wirkstoffsalz in einer Zweiphasenmatrix enthält. Die Zweiphasenmatrix besteht aus einem hydrophoben Matrixpolymer mit einem darin dispergierten Silikat zur Aufnahme des hydrophilen Arzneistoffsalzes, wobei zusätzlich hydrophobe Lösemittel verwendet werden. Die Herstellung der Matrix erfolgt durch Trocknen der Dispersion bei 70°C. Der Gehalt an Rotigotin in der Matrix beträgt 2-5 Gew%.

Dieses System weist jedoch eine Reihe von Nachteilen auf:
- Die Herstellung ist mehrstufig und aufwendig. Das Wirkstoffsalz muß gelöst, dann mit dem Silikat gemischt werden, dann mit einem Emulgator vermengt werden, um die Lösung schließlich mit dem in einem organischen Lösungsmittel - üblicherweise Heptan, Ethylacetat oder Toluol - gelösten Matrixpolymer, z.B. in einem Silikonkleber, zu emulgieren.
- Die resultierende Emulsion ist schwierig zu handhaben.
- Die Wirkstoffbeladung ist begrenzt durch die Löslichkeit des Rotigotins im jeweiligen Lösungsmittelsystem. Zudem findet beim Entfernen der Lösungsmittel während der Herstellung eine Aufkonzentrierung statt, bei der es zu einer unerwünschten Kristallbildung kommen kann. Auch hierdurch ist die maximale Menge an Wirkstoff, die sich in die Matrix einarbeiten läßt, beschränkt. Eine niedrige Wirkstoffbeladung wiederum begrenzt die Freisetzungskapazität der Matrix pro Zeiteinheit und/oder ihre funktionale Lebenszeit.
- Das im Pflaster verbleibende Silikat bzw. Siliziumdioxid stellt eine Diffusionsbarriere für den Wirkstoff dar, die die Freigabe des Wirkstoffes negativ beeinflussen kann.
- Das anorganische Silikat beeinflußt die Wasseraufnahme des Pflasters. Porenbildung durch Herauslösen wasserlöslicher Matrixbestandteile an der Grenzfläche zur Haut können zu einer schlecht kontrollierbaren Freisetzung des Wirkstoffes führen.

WO 99/49852 beschreibt ein Transdermales Therapeutisches System (TTS) enthaltend ein Haftklebesystem, das auf Acrylat oder Silikon basiert und in dem Rotigotin in Form der freien Base vorliegt. Das offenbarte TTS ermöglicht therapeutisch relevante Fluxraten von Rotigotin durch Humanhaut.

Rotigotin ist in hydrophoben Polymeren, wie z.B. in Silikon, nur schwach löslich. Aus diesen Gründen wird in WO 99/49852 die Zugabe von Zusatzstoffen zur Verbesserung des Lösungsverhaltens des Rotigotins vorgeschlagen. Dabei handelt es sich insbesondere um hydrophile Polymere, wie z.B. Polyvinylpyrrolidon (PVP), Copolymere von Vinylpyrrolidon und Vinylacetat, Polyethylenglycol, Polypropylenglycol, Copolymere aus Ethylen und Vinylacetat sowie um Glycerin und dessen Ester.

WO 02/089778 und WO 02/089777 beschreiben ebenfalls ein Lösemittel-basiertes transdermales System zur Verabreichung von Rotigotin. Gemäß WO 02/089778 und WO 02/089777 werden ebenfalls oberflächenaktive Substanzen oder amphiphile Substanzen als Kristallisationsinhibitor zugesetzt.

EP 1 256 340 beschreibt ebenfalls ein Lösemittel-basiertes transdermales System zur Verabreichung von Rotigotin. Die Matrix enthält neben einer Silikon-mischung z.B. den Kristallisationsinhibitor Polyvinylpyrrolidon.

WO 2004/012721 offenbart ein transdermales System eines mit Rotigotin-Base übersättigten Matrixpolymers, welches frei von Löslichkeitsvermittlern, Kristallisationsinhibitoren und Dispersionsmitteln ist.

Es war nun die Aufgabe der vorliegenden Erfindung eine Matrix zur Verfügung zu stellen, die einfach aufgebaut ist und möglichst wenige Hilfsstoffe enthält, die aber dennoch die Verabreichung von Rotigotin durch die Haut in therapeutisch relevanten Fluxraten erlaubt, lagerstabil ist und die Einarbeitung von Rotigotin-Base in einem weiten Konzentrationsbereich ermöglicht.

### Abbildungen:

Abbildung 1 zeigt eine mikroskopische Aufnahme von amorphen Rotigotin-Partikeln in einer Silikonmatrix, die gemäß Ausführungsbeispiel 2b (Vergleichsbeispiel) im Lösemittelverfahren ohne Dispersionsmittel hergestellt wurde.

Abbildung 2 zeigt mikroskopische Aufnahmen von amorphen Rotigotin-Partikeln in einer erfindungsgemäßen Silikonmatrix, die gemäß Ausführungsbeispiel 1 durch "tempern" ohne Dispersionsmittel hergestellt wurde.

Abbildung 3 zeigt den Vergleich von in-vitro Rotigotin-Fluxraten, die nach Auftrag einer erfindungsgemäßen Vorrichtung (Charge 20204071), einer gemäß Ausführungsbeispiel 2b im Lösemittelverfahren ohne Dispersionsmittel hergestellten Vergleichscharge (Charge 20204074) und einer in WO 99/49852 beschriebenen Vorrichtung (Charge 20107012) auf Mäusehaut erzielt werden.

Abbildung 4 zeigt den Vergleich von in-vitro Rotigotin-Fluxraten, die nach Auftrag einer erfindungsgemäßen Vorrichtung (Charge 20204071) und einer in WO 99/49852 beschriebenen Vorrichtung (Charge WE11682) auf Humanhaut erzielt werden.

Abbildung 5 zeigt beispielhaft den Aufbau eines monolithischen TTS mit einer wirkstoffhaltigen Matrix (1), einer wirkstoffundurchlässigen Rückschicht (2) und einer vor Gebrauch entfernbaren Schutzschicht (3).

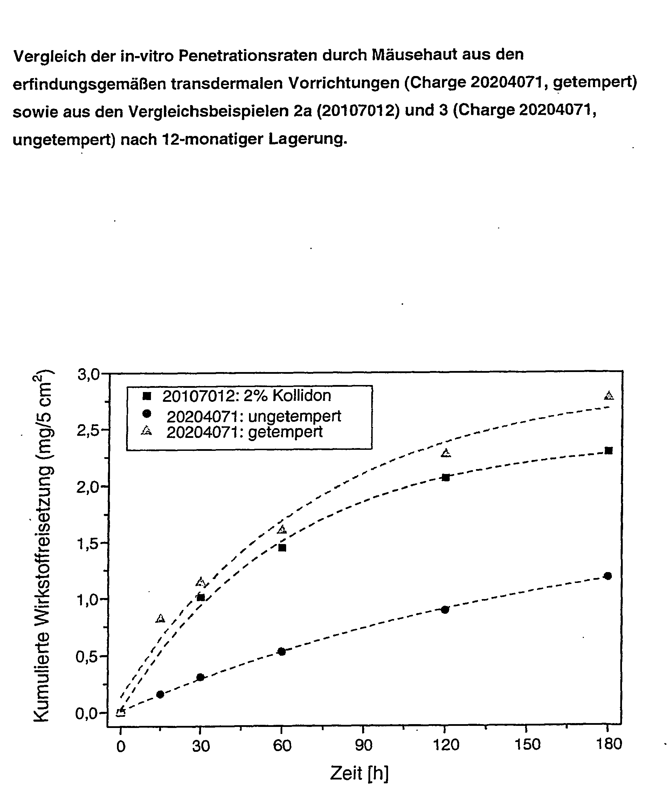

Abbildung 6 zeigt einen Vergleich der in-vitro Penetrationsraten durch Mäusehaut aus den erfindungsgemäßen transdermalen Vorrichtungen (Charge 20204071, getempert) sowie aus den Vergleichsbeispielen 2a (20107012) und 3 (Charge 20204071, ungetempert) nach 12-monatiger Lagerung.

### Beschreibung der Erfindung

Rotigotin-Base liegt als Feststoff in Form von Kristallen vor, die in den zur Lösung von Matrixpolymeren geeigneten Lösemitteln, z.B. Hexan, Ethylacetat und Toluol, nahezu unlöslich sind.

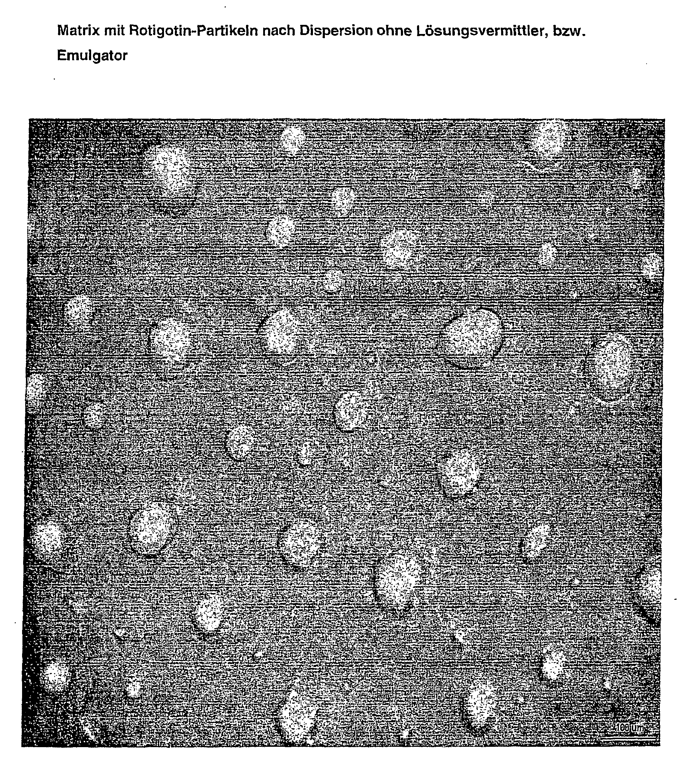

Zur Herstellung einer rotigotinhaltigen Matrix werden die Rotigotin-Kristalle daher nach dem Stand der Technik zunächst in Lösemittel, z.B. Ethanol, gelöst und sodann zur Polymerphase, z.B. in Hexan, gegeben. Zur Herstellung einer Feindispersion der wirkstoffhaltigen Phase in der Polymerphase werden Dispersionsmittel, wie z.B. die in WO 99/49852 genannten hydrophilen Polymere, verwendet. Werden bei diesem Verfahren die Dispersionsmittel nicht wie vorgeschlagen, zugesetzt, können sich große Wirkstoffinseln bilden (Abbildung 1). Letztere bergen dann die Gefahr der Hautreizung, der Wirkstoff-Rekristallisation, der verringerten Adhäsion der Klebermatrix und der Schwankung der Wirkstoffbeladung.

Es wurde nun überraschenderweise festgestellt, dass dennoch auf die Verwendung eines zusätzlichen Löse- oder Dispersionsmittels bzw. Kristallisationsinhibitors verzichtet werden kann, wenn auf das Vorlösen des Rotigotins in Lösemittel, z.B. in Ethanol, vor dem Einbringen in eine Matrix, z.B. in eine Silikonmatrix, verzichtet wird.

In einer Ausführungsform der Erfindung wird beispielsweise die Rotigotin-Base in kristalliner Form in eine Lösung eines Silikonpolymers, z.B. eines aminoresistenten Silikonhaftklebers, in Heptan, Toluol oder Ethylactetat eingerührt, die Mischung auf eine Folie, z.B. eine silikonisierte Polyesterfolie, beschichtet und das Lösemittel durch Trocknen bei 50°C entfernt. Anschließend wird die Matrix auf eine Temperatur oberhalb des Schmelzpunkts von Rotigotin, d.h. oberhalb von ca. 74°C, solange erwärmt ("getempert"), bis die Rotigotin-Kristalle geschmolzen sind. Schließlich wird auf Raumtemperatur abgekühlt. Das Rotigotin liegt dann in Form amorpher Partikel oder Tröpfchen feinverteilt in der silikonbasierten Matrix vor.

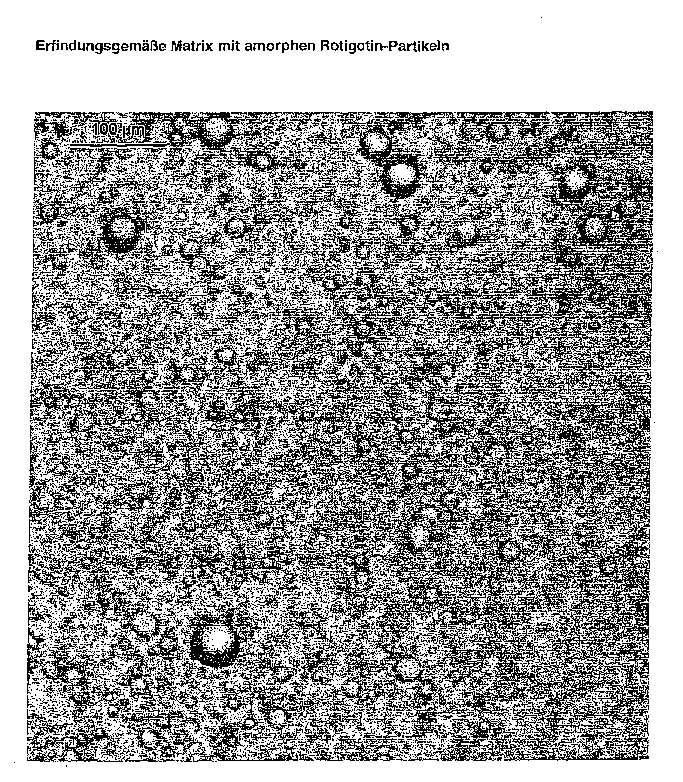

Bei mikroskopischer Betrachtung zeigte sich, dass die amorphen Rotigotin-Partikel in der Silikonmatrix überraschend feinverteilt sind und eine Größe von maximal etwa 30-40 µm, in der Mehrzahl aber kleiner als 20 µm aufweisen (Abbildung 2). Selbst nach sechsmonatiger Lagerung bei Raumtemperatur zeigten die amorphen Rotigotin-Partikel in der Silikonmatrix keine Tendenz zur Rekristallisierung.

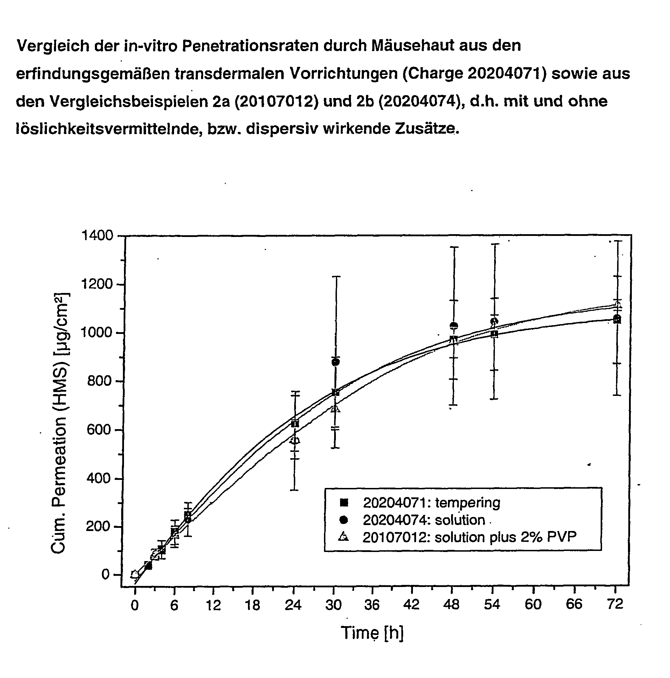

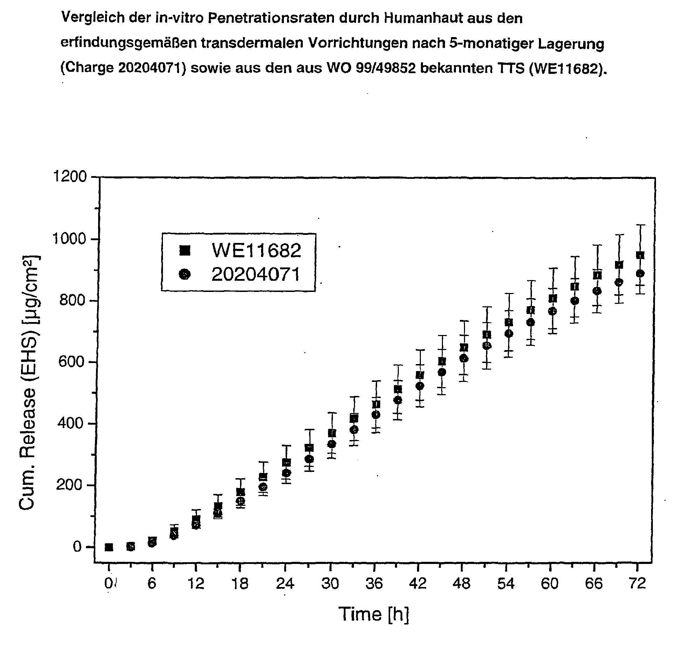

Ferner zeigte sich in in-vitro Permeationsexperimenten an Mäusehaut und Humanhaut, dass transdermale Systeme, die die erfindungsgemäß hergestellten, die amorphen Rotigotin-Partikel enthaltenden Silikon-Matrices enthalten, beim Auftrag auf die Haut zu Rotigotin-Permeationsraten führen, die mit den im Lösemittelverfahren gemäß WO 99/49852 hergestellten, therapeutisch einsetzbaren TTS nahezu identisch sind (Abbildungen 3 und 4). Auch nach fünfmonatiger Lagerung bei Raumtemperatur war das Freisetzungsverhalten der erfindungsgemäßen TTS unverändert (Abbildung 4).

Dies bedeutet, dass der Zusatz eines Lösungsvermittlers/Dispersionsmittels zur Erreichung einer pharmakologisch relevanten Fluxrate von Rotigotin aus Polymermatrices erfindungsgemäß nicht erforderlich ist.

Vielmehr können überraschenderweise mit einer sehr einfach aufgebauten Matrix therapeutisch relevante Fluxraten erzielt werden, wenn das nicht im Matrixpolymer gelöste Rotigotin feinverteilt in amorphen Partikeln in der Matrix "konserviert" werden kann.

Gelingt dies, indem beispielsweise die kristalline Wirkstoff-Form durch Erhitzung der mit Rotigotin übersättigten Matrix in die amorphe Form überführt wird, die dann in der Matrix feinverteilt dispergiert vorliegt, ist der Zusatz von Löslichkeitsvermittlern, Kristallisationsinhibitoren und/oder Dispersionsmittel, z.B. in Form polarer innerer-Phase Polymere, nicht erforderlich.

Da die erfindungsgemäßen übersättigten, vorzugsweise silikonbasierten Matrices keine potentiell peroxidhaltigen hydrophilen Polymere, wie PVP, enthalten, kann auch auf den Zusatz von Additiven zur Peroxidbeseitigung ("Peroxidfängern") verzichtet werden.

Ferner enthält die Matrix auch keine anorganischen Silikate oder Hautpenetrationsverbesserer ("Enhancer").

Auch nach 12-monatiger Lagerung zeigen die erfindungsgemäßen TTS keine Zeichen der Rotigotin-Rekristallisierung oder eine Veränderung der Partikelgröße. Zudem zeigte sich in in-vitro Freisetzungsexperimenten bei den erfindungsgemäßen TTS ein unverändertes und mit dem gemäß Beispiel 2a hergestellten, Kollidon-haltigen TTS vergleichbares Freisetzungsprofil. Im Gegensatz dazu lieferte ein gemäß Ausführungsbeispiel 3 hergestelltes, kristallines Rotigotin enthaltendes TTS, bei dem auf den Schritt-des Erhitzens über den Schmelzpunkt von Rotigotin verzichtet wurde, eine deutlich geringere Wirkstofffreisetzung.

Schließlich kann auch auf den Einsatz von in Heißschmelzverfahren üblichen Weichmachern zur Senkung der dynamischen Viskosität von Matrixpolymeren verzichtet werden, da das Polymer im Lösemittelverfahren verarbeitet wird.

Ein Gegenstand der Erfindung ist daher eine Matrix zur transdermalen Verabreichung von Rotigotin [(-)-5,6,7,8-Tetrahydro-6-[propyl[2-(2-thienyl)ethyl]amino]-1-naphtol], enthaltend ein mit Rotigotin-Base übersättigtes Matrixpolymer, dadurch gekennzeichnet, dass der nicht im Matrixpolymer gelöste Anteil des Rotigotins als amorphe Partikel mit einem mittleren Durchmesser von maximal 30 µm im Matrixpolymer dispergiert ist und die Matrix frei von Löslichkeitsvermittlern, Kristallisationsinhibitoren und Dispersionsmittein ist, mit der Maßgabe, dass die Matrix keinen heißschmelzfähigen Haftkleber enthält.

Ein weiterer Gegenstand der Erfindung ist eine Rotigotin [(-)-5,6,7,8-Tetrahydro-6-[propyl[2-(2-thienyl)ethyljamino]-1-naphtol]-haltige Matrix bestehend aus
(a) Matrixpolymer,
(b) Rotigotin-Base in einer Konzentration oberhalb der Löslichkeitsgrenze des Matrixpolymers, wobei der nicht im Matrixpolymer gelöste Anteil des Rotigotins als amorphe Partikel mit einem mittleren Durchmesser von maximal 30 µm im Matrixpolymer dispergiert ist und
(c) optional einem oder mehreren Antioxidanzien, mit der Maßgabe, dass die Matrix keinen heißschmelzfähigen Haftkleber enthält.

Die erfindungsgemäße Matrix enthält im allgemeinen mindestens 60 Gew%, bevorzugt 70-95 Gew%, besonders bevorzugt 80-91 Gew% Matrixpolymer, jeweils bezogen auf das Matrixgewicht.

In einer bevorzugten Ausführungsform der Erfindung ist das Matrixpolymer ein Silikon, vorzugsweise ein amino-resistentes Silikon oder eine Silikon-Mischung.

Ein weiterer Gegenstand der Erfindung ist daher eine Rotigotin [(-)-5,6,7,8-Tetrahydro-6-[propyl[2-(2-theinyl)ethyl]amino]-1-naphtol]-haltige Matrix bestehend aus
(a) amino-resistentem Silikon,
(b) Rotigotin-Base in einer Konzentration oberhalb der Löslichkeitsgrenze des Silikons, wobei der nicht im Silikon gelöste Anteil des Rotigotins als amorphe Partikel mit einem mittleren Durchmesser von maximal 30 µm im Silikon dispergiert ist und
(c) optional einem oder mehreren Antioxidanzien, mit der Maßgabe, dass die Matrix keinen heißschmelzfähigen Haftkleber enthält.

Unter dem Begriff "Matrix" wird in dieser Patentanmeldung eine pharmazeutische Formulierung verstanden, die mindestens ein Matrixpolymer umfasst und die ein disperses System bilden kann, mit der Maßgabe, dass die Matrix keinen heißschmelzfähigen Haftkleber enthält.

Unter dem Begriff "Rotigotin-Base" wird in dieser Anmeldung verstanden, dass weniger als 5 Gew%, bevorzugt weniger als 2 Gew%, besonders bevorzugt weniger als 1 Gew% des Rotigotins in Salzform vorliegt.

Unter dem Begriff "Partikel" werden in dieser Patentanmeldung mikroskopisch sichtbare Rotigotin-Ansammlungen, z.B. in Tröpfchen-Form, in der Matrix verstanden.

Unter dem Begriff "mittlerer Durchmesser" wird der Durchschnittswert aller Durchmesser (jeweils in den Dimensionen x,y,z) der in einer gegebenen Matrix vorliegenden Rotigotin-Partikel verstanden. Dies kann bestimmt werden, indem die Rotigotin-haltige Matrix mit einem Mikroskop untersucht und das Bild mit der Software Nikon LuciaDi ausgewertet wird.

Unter dem Ausdruck "mit Rotigotin übersättigte Matrix" wird in dieser Patentanmeldung verstanden, dass zumindestens ein Teil des Rotigotins nicht in im Polymer gelöster Form, sondern als Partikel in der Matrix dispergiert vorliegt.

Unter dem Begriff "Matrixpolymer" werden die einem pharmazeutischen Fachmann zur Herstellung von transdermalen Arzneiformen geläufigen Polymere verstanden. Beispiele hierfür sind Silikone, Ethylvinylacetate (EVA), Styrol-Block-Copolymere (SXS), Acrylate und Methacrylate, Polyurethane, Vinylacetate sowie Gummen, insbesondere Polyolefine und Polyterpene, z.B. Polyisobutylene, Polybutadiene, Neoprene oder Polyisoprene sowie geeignete Mischungen dieser Matrixpolymere.

Unter dem Ausdruck "silikonbasierte Matrix" wird in dieser Patentanmeldung eine Matrix verstanden, die mindestens 60 Gew%, bevorzugt 70-95 Gew%, besonders bevorzugt 80-91 Gew% Silikon, bezogen auf das Matrixgewicht, enthält.

In einer bevorzugten Ausführungsform der Erfindung werden als Matrixpolymere solche verwendet, in denen Rotigotin eine Löslichkeit von weniger als 5 Gew%, besonders bevorzugt weniger als 3 Gew% und ganz besonders bevorzugt weniger als 1 Gew% hat.

Die mit Rotigotin übersättigte Matrix kann zur Verarbeitung in verschiedenen galenischen Arzneiformen verwendet werden. Die rotigotinhaltige Matrix kann dabei als adhäsive (selbstklebende) oder als nicht-adhäsive Matrix ausgebildet sein.

Bevorzugt liegen die amorphen Rotigotin-Partikel in einer selbstklebenden Matrix, besonders bevorzugt in einer selbstklebenden Silikonhaftklebermatrix dispergiert vor.

Bevorzugte Silikonhaftkleber zur Verwendung in der erfindungsgemäßen selbstklebenden Silikonhaftklebermatrix sind aminoresistente, drucksensitive Polyorganosiloxankleber.

Silikonhaftkleber stellen in den meisten Fällen Polydimethylsiloxane dar, allerdings können prinzipiell statt Methylgruppen auch andere organische Reste, wie z.B. Ethyl- oder Phenylgruppen vorhanden sein. Aminoresistente Silikonhaftkleber zeichnen sich im allgemeinen dadurch aus, dass sie keine oder nur wenige freie Silanolfunktionen enthalten, da die Si-OH-Gruppen alkyliert wurden. Solche Kleber sind in der Patentschrift EP 180 377 beschrieben.

Besonders bevorzugte Kleber sind Kondensate oder Mischungen von Silikonharzen und Polyorganosiloxanen, wie beispielsweise in US RE 35 474 beschrieben.

Geeignete Polyorganosiloxankleber sind kommerziell bei Dow Corning als sogenannte BIO-PSA-Haftkleber erhältlich. Besonders geeignet sind Haftkleber, die von Dow Corning unter der Bezeichnung Bio-PSA 7-4201 und Bio-PSA 7-4301 vertrieben werden, sowie geeignete Mischungen dieser Kleber. Diese Mischungen von Silikonklebern mit starker und mittlerer Haftkraft ("tack"), insbesondere Mischungen im Verhältnis 40:60 bis 60:40 von Bio-PSA 7-4201 und Bio-PSA 7-4301, zeichnen sich durch eine besonders günstige Adhesions/Cohesionsbalance aus.

Die Wirkstoffkonzentration der erfindungsgemäßen Matrix unterliegt nicht den verfahrensbedingten Beschränkungen wie die im Lösungsmittelverfahren nach dem Stand der Technik hergestellten Matrices.

Da im Verfahren nach dem Stand der Technik die kristalline Rotigotin-Base in Ethanol vorgelöst wird, ist die Wirkstoffbeladung durch die Löslichkeit des Rotigotins im verwendeten Lösemittel begrenzt. Eine Matrixbeladung mit mehr als etwa 15 Gew% Rotigotin ist daher im bekannten Lösemittelverfahren schwierig. Diese Beschränkung entfällt bei den erfindungsgemäß hergestellten Matrices, da ein Vorlösen der Rotigotin-Base in Ethanol nicht erforderlich ist.

Aus diesem Grund ist auch die Inkorporation von Rotigotin-Base in Konzentrationen oberhalb von 15 Gew% möglich. Dies ist zum Beispiel besonders hilfreich, wenn eine längere Rotigotin-Freisetzung aus der Matrix zum Beispiel über 5, 6 oder 7 Tage gewünscht ist.

Die Wirkstoffkonzentration in der Matrix kann prinzipiell zwischen 1 und etwa 40 Gew%, bezogen auf das Gesamtgewicht der Matrix, liegen, wobei Rotigotin-Konzentrationen zwischen 5 und 30 Gew% und besonders zwischen 7 und 25 Gew% bevorzugt werden.

Für eine 7 Tage dauernde Freisetzung von Rotigotin aus der Matrix wird eine Rotigotin-Konzentration in der Matrix von mindestens 15 Gew%, besonders von mindestens 20 Gew% bevorzugt.

Antioxidanzien werden bevorzugt in einer Gesamtkonzentration bis 2 Gew%, bevorzugt 0,05-0,5 Gew% (bezogen auf das Matrixgewicht) zugesetzt. Bevorzugte Beispiele sind Alpha-Tocopherol, Ascorbylpalmitat und Mischungen davon.

In einer bevorzugten Ausführungsform der Erfindung besteht die erfindungsgemäße Matrix aus
(a) 60-95 Gew% wenigstens eines Matrixpolymers, bevorzugt eines Silikons oder Silikongemischs,
(b) 1-40 Gew%, bevorzugt 5-30 Gew%, besonders bevorzugt 7-20.Gew% im Matrixpolymer dispergierte amorphe Rotigotin-Base, wobei der nicht im Silikon gelöste Teil des Rotigotins in Form amorpher Partikel mit einem mittleren Durchmesser von maximal 30 µm im Silikon dispergiert ist und
(c) 0-2 Gew%, bevorzugt 0,05-0,5 Gew% Antioxidans, mit der Maßgabe, dass die Matrix keinen heißschmelzfähigen Haftkleber enthält.

Die Größenverteilung der Rotigotin-Partikel in der mit Rotigotin übersättigten, bevorzugt silikonbasierten Matrix sollte möglichst gleichmäßig sein, wobei der mittlere Durchmesser bevorzugt unter 25 µm, besonders bevorzugt unter 20 µm liegen sollte.

In einer bevorzugten Ausführungsform ist die erfindungsgemäße Matrix Bestandteil einer Vorrichtung, insbesondere einer flächenförmigen Vorrichtung, zur transdermalen Verabreichung von Rotigotin, wobei die Vorrichtung weitere Bestandteile, wie z.B. eine Schutzschicht, eine Rückschicht, weitere Polymerschichten und/oder eine die Wirkstoffabgabe kontrollierende Membran.enthalten kann.

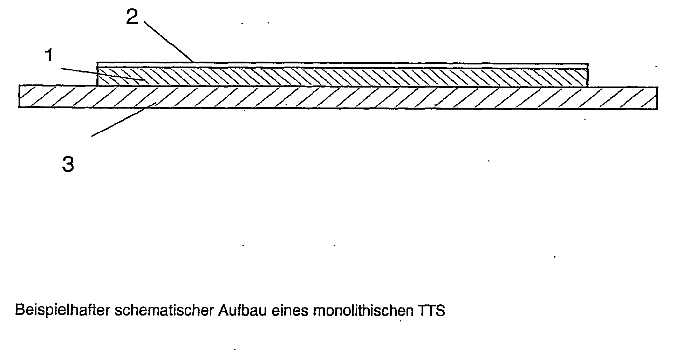

In einer besonders bevorzugten Ausführungsform der Erfindung ist die erfindungsgemäße Vorrichtung als sogenanntes monolithisches Pflaster ausgestaltet, das heißt, sie besteht aus einer für den Wirkstoff undurchlässigen Rückschicht (2), einer selbstklebenden, mit Rotigotin übersättigten, vorzugsweise silikonbasierten Matrix (1), in der die freie Base von Rotigotin in amorpher Form dispergiert ist und die keinen Löslichkeitsvermittler enthält sowie einer vor dem Auftrag auf die Haut des Patienten ablösbaren Schicht (3), wie in Abbildung 5 dargestellt.

In anderen Ausführungsformen der Erfindung kann das Rotigotin auch in einer nichtadhäsiven, übersättigten, vorzugsweise silikonbasierten Matrix vorliegen. Die flächenförmige Vorrichtung kann dann eine zusätzliche wirkstoffreie Kleberschicht oder ein sogenanntes "overtape" aufweisen.

Ein Gegenstand der Erfindung ist daher eine flächenförmige Vorrichtung zur transdermalen Verabreichung von Rotigotin [(-)-5,6,7,8-Tetrahydro-6-[propyl[2-(2-theinyl)ethyl]amino]-1-naphtolj, enthaltend eine Rotigotin-haltige Matrixschicht und eine für den Wirkstoff undurchlässige Rückschicht, dadurch charakterisiert, dass die Matrixschicht besteht aus
(a) Matrixpolymer, vorzugsweise einem amino-resistenten Silikon oder einer Silikon-Mischung,
(b) Rotigotin-Base in einer Konzentration oberhalb der Löslichkeitsgrenze des Matrixpolymers, wobei der ,nicht im Matrixpolymer gelöste Anteil des Rotigotins als amorphe Partikel mit einem mittleren Durchmesser von maximal 30 µm im Matrixpolymer dispergiert ist und
(c) optional einem oder mehreren Antioxidanzien, mit der Maßgabe, dass die Matrix keinen heißschmelzfähigen Haftkleber enthält.

In einer bevorzugten Ausführungsform der Erfindung ist die flächenförmige Vorrichtung als monolithisches System aufgebaut und enthält eine selbstklebende Rotigotin-haltige Matrixschicht, die auf einem aminoresistenten Silikonhaftkleber basiert.

Die Oberfläche der Vorrichtung kann zwischen 5 und ca. 80 cm² groß sein, liegt bevorzugt zwischen 10 und 60 cm² und besonders bevorzugt zwischen 20 und 40 cm².

Die Dicke der Matrixschicht in den erfindungsgemäßen Vorrichtungen liegt üblicherweise im Bereich 40-300 µm, wobei Matrixstärken von 50-200 µm und besonders von 70-150 µm bevorzugt werden. Daraus ergibt sich ein bevorzugtes Matrixgewicht von ca. 40-200 g/m².

Bevorzugte Rotigotin-Konzentrationen in der Matrixschicht der Vorrichtung liegen zwischen 5 und 30 Gew% und besonders bevorzugt zwischen 7 und 25 Gew%, bezogen auf das Gesamtgewicht der Matrix. Ist die Vorrichtung für eine mehr als 5-tägige Applikation vorgesehen, sind in der Regel Konzentrationen des Rotigotins von mehr als 15 Gew%, bevorzugt mehr als 20 Gew% erforderlich. Typische Konzentrationen für 7 Tage-Pflaster liegen bei 20-30 Gew%.

Der Beladungsgrad der Matrix in der erfindungsgemäßen Vorrichtung liegt dabei grundsätzlich zwischen 0.1 und 9 mg Rotigotin/cm² Matrixoberfläche. Der bevorzugte Beladungsgehalt liegt im Bereich 0.3 - 6 mg Rotigotin/cm². Für Vorrichtungen zur täglichen oder 2-tägigen Verabreichung besonders bevorzugt wird eine Rotigotin-Beladung zwischen 0.3 und 1,5 mg Rotigotin/cm², bei 7-Tage-Systemen bei 2,5-6,0 mg/cm².

Die nachfolgende Tabelle zeigt Wirkstoffkonzentration und Matrixgewicht der für die Hautpermeationsexperimente (Fig. 2, 3) eingesetzten monolithischen Pflaster.

| Chargennummer | Herstellbedingung | Wirkstoff-Konzentration | Matrixgewicht (g/m²) | Kumulativer Flux durch Humanhaut µg/cm²/72h | Kumulativer Flux durch Mäusehaut µg/cm²/72h |
|---|---|---|---|---|---|
| 20204071 | Getempert 90°C, 75 Min | 8,87 Gew% | 129 | 850 | 1030 |
| 20107012 | Lösungsmittel-verfahren¹ | 9 Gew% | 110 | n.b. | 1080 |
| WE 11682 | Lösungsmittel-verfahren¹ | 9 Gew% | 50 | 900 | n.b. |

| | | | | | |
|---|---|---|---|---|---|
| ¹= Vergleichsbeispiel entsprechend WO 99/49852; siehe Ausführungsbeispiel 2a n.b.=nicht bestimmt | | | | | |

Die Größenverteilung der Rotigotin-Partikel in der silikonbasierten Matrix der erfindungsgemäßen Vorrichtungen sollte möglichst gleichmäßig sein und im Mittel unter 30 µm liegen, wobei der mittlere Durchmesser bevorzugt unter 25 µm, besonders bevorzugt unter 20 µm liegen sollte.

Bevorzugt sollten zudem in einer gegebenen Matrixschicht keine Partikel vorhanden sein, deren Durchmesser in der größten Dimension (x,y,z) größer ist als 90% der Dicke der jeweiligen Matrixschicht.

Die Rückschicht, auf die die Matrixmasse der erfindungsgemäßen Vorrichtung ausgestrichen wird, sollte für die Inhaltsstoffe der Matrix inert und für Rotigotin undurchlässig sein. Geeignete Materialien sind beispielsweise Polyester, Polyamide, Polyethylene, Polyproylene, Polyurethane, PVC oder Kombinationen dieser Materialien. Die Folien können silikonsiert sein und/oder mit einer Aluminiumschicht versehen sein. Die Dicke variiert üblicherweise zwischen 10 und 100 µm und liegt bevorzugt zwischen 20 und 40 µm.

Die Vorrichtung enthält weiterhin bevorzugt eine Schutzschicht oder Folie, die unmittelbar vor Gebrauch der Vorrichtung, das heißt vor dem Auftrag auf die Haut, entfernt wird. Diese Schutzschicht kann beispielsweise aus Polyester, Polyethylen oder Polypropylen bestehen. Diese Schicht kann zusätzlich mit Aluminium oder Fluorpolymeren beschichtet sein. Die Dicke dieser Schutzschicht liegt üblicherweise zwischen 30 und 200 µm. Zur besseren Entfernung, der Schutzschicht unmittelbar vor Gebrauch besteht die Schutzschicht bevorzugt aus zwei getrennten Folien, deren Enden überlappen können. Entsprechende Ausgestaltungen sind von konventionellen Pflastern bekannt.

Rotigotin ist ein Dopamin-Agonist. Die erfindungsgemäßen Matrices und Vorrichtungen sind daher insbesondere zur Behandlung von Erkrankungen, die mit einem gestörten Dopamin-Stoffwechsel einhergehen, geeignet.

Ein Gegenstand der Erfindung ist daher die Verwendung einer erfindungsgemäßen Vorrichtung oder einer erfindungsgemäßen Matrix in einem Medikament zur Behandlung von Morbus Parkinson, Restless Leg oder Depressionen.

Die erfindungsgemäße mit Rotigotin übersättigte, vorzugsweise silikonbasierte Matrix kann in einfacher Weise hergestellt werden, indem die Rotigotin-Base in kristalliner Form in eine Lösung eines entsprechenden Matrixpolymers eingerührt wird, das Lösemittel durch Trocknen bei 50°C entfernt und schließlich die lösungsmittelfreie Matrix auf eine Temperatur oberhalb des Schmelzpunkts von Rotigotin, d.h. oberhalb von ca 74°C, solange erhitzt ("getempert") wird, bis die Rotigotin-Kristalle geschmolzen sind. Anschließend wird auf Raumtemperatur abgekühlt, so dass das Rotigotin schließlich in Form amorpher Partikel oder Tröpfchen in der erfindungsgemäßen Matrix vorliegt. Der Abkühlschritt wird bevorzugt "passiv" durchgeführt, das heißt, die Rotigotin-haltige Matrix wird Raumtemperatur ausgesetzt; eine zusätzliche Kühlung ist in der Regel nicht erforderlich.

Ein Gegenstand der Erfindung ist daher ein Verfahren zur Herstellung einer Matrix zur transdermalen Verabreichung von Rotigotin, gekennzeichnet durch die aufeinanderfolgenden Schritte:
(a) Auflösen des Matrixpolymers, z.B. des Silikons, in einem Lösemittel, z.B. in Heptan, Ethylacetat und Toluol,
(b) Zugabe von Rotigotin-Base in kristalliner Form in einer Menge oberhalb der Löslichkeitsgrenze des Polymers,
(c) Entfernen des Lösemittels und Erwärmen der hergestellten Matrixmasse auf eine Temperatur von mindestens 74°C bis das Rotigotin in der Matrixmasse geschmolzen ist,
(d) Abkühlen, bevorzugt passives Abkühlen der Matrixmasse, mit der Maßgabe, dass die Matrix keinen heißschmelzfähigen Haftkleber enthält.

Dabei kann in Schritt (c) die Entfernung des Lösemittels und das Schmelzen des Rotigotins durch kontinuierliche Temperaturerhöhung, z.B. von 50°C auf 90°C, beispielsweise in einer Trockenstrasse, realisiert werden.

Alternativ kann in Schritt (c) zunächst in einem Schritt (c1) das Lösemittel bei einer Temperatur von 40-60 °C entfernt und die lösemittelfreie Matrix in einem Schritt (c2) sodann auf mindestens 74°C erwärmt werden, bis das Rotigotin geschmolzen ist.

Geeignete Prozesstemperaturen für das Schmelzen von Rotigotin liegen beispielsweise bei 75-120°C, bevorzugt bei 80-100°C, besonders bevorzugt bei 90°C.

Soll eine erfindungsgemäße Vorrichtung hergestellt werden, die neben der Rotigotin-haltigen Matrix eine Wirkstoff-undurchlässige Rückschicht aufweist, so wird die bei der oben beschriebenen Matrixherstellung in Schritt (b) entstehende Rotigotin-haltige Polymermasse vor der Entfernung des Lösemittels auf eine geeignete Folie, z.B. eine Polyesterfolie ausgestrichen.

Ein Gegenstand der Erfindung ist daher ein Verfahren zur Herstellung einer flächenförmigen Vorrichtung zur transdermalen Verabreichung von Rotigotin, umfassend eine Rotigotin-haltige Matrix, gekennzeichnet durch die aufeinanderfolgenden Schritte:
(a) Auflösen des Matrixpolymers, z.B. des Silikons, in einem Lösemittel,
(b) Zugabe von Rotigotin-Base in kristalliner Form in einer Menge oberhalb der Löslichkeitsgrenze des Polymers,
(c) Ausstreichen der Rotigotin-haltigen Polymermasse auf einer geeigneten Folie,
(d) Entfernen des Lösemittels und Erwärmen der hergestellten Matrixmasse auf eine Temperatur von mindestens 74°C, bis das Rotigotin in der Matrixmasse geschmolzen ist,
(e) Abkühlen, bevorzugt passives Abkühlen, der Matrixmasse, mit der Maßgabe, dass die Matrix keinen heißschmelzfähigen Haftkleber enthält.

Dabei kann die Entfernung des Lösemittels und das Schmelzen des Rotigotins gemäß Schritt (d) entweder durch kontinuierliche Temperaturerhöhung z.B. von 50°C auf 90°C erfolgen oder aber stufenweise in zwei separaten Schritten (d1) und (d2) erfolgen, wie bereits weiter oben beschrieben.

Vor Zugabe des kristallinen Rotigotins können die in der Regel nadelförmigen Rotigotin-Kristalle gegebenenfalls durch geeignete Vorbehandlung, z.B. durch Mahlen oder Zerstossen und anschliessendes Sieben auf die gewünschte Größe, z.B. 50 µm Länge, reduziert werden.

### Experimenteller Teil:

### 1. Herstellung einer erfindungsgemäßen Silikon-basierten Vorrichtung

1,8 g kristallines Rotigotin (freie Base) wurden gemahlen und als Puder mit einer Korngröße unter 40 µm zu einer 74%igen (g/g) Lösung von Silikon-Polymeren in Heptan (entspricht 9 g Bio-PSA 7-4201 und 9 g BIO-PSA 7-4301) gegeben. Die Mischung wurde zur Herstellung einer homogenen Dispersion mit einem Ultraturrax bei 10 000 UpM für 1 Minute gerührt. Anschließend wurde die Rotigotin-haltige Silikonmasse auf eine Scotch Pak 1109 Folie ausgestrichen (6 mm/Sec) und für 30 Minuten bei 50°C getrocknet. Schließlich wurde Schutzfolie (MN 19) aufgetragen.

Anschließend wurde für 75 Minuten auf 90°C erhitzt.

### 2. Veraleichsbeispiele: Herstellung der silikonbasierten Matrix im Lösunasmittelverfahren nach dem Stand der Technik mit (Beispiel 2a) oder ohne (Beispiel 2b) Zusatz von PVP

1,8 g kristallines Rotigotin (freie Base) wurden gemahlen und mit oder ohne 2,4 g Kollidon (PVP) in 4 g Ethanol (96%) gelöst zu einer 74%igen (g/g) Lösung von Silikon-Polymeren in Heptan (entspricht einem Gemisch von 9 g Bio-PSA 7-4201 und 9 g BIO-PSA 7-4301) gegeben. Die Mischung wurde zur Herstellung einer homogenen Dispersion mit einem Ultraturrax bei 10 000 UpM für 1 Minute gerührt. Anschließend wurde die Rotigotin-haltige Silikonmasse auf eine Scotch Pak 1109 Folie ausgestrichen (6 mm/Sec) und für 30 Minuten bei 50°C getrocknet. Schließlich wurde Schutzfolie (MN 19) aufgetragen.

### 3. Beispiel: Herstellung einer Silikon-basierten Matrix ohne Vorlösen und Tempern

1,8 g kristallines Rotigotin (freie Base) wurden gemahlen und als Puder mit einer Korngröße unter 40 µm zu einer 74%igen (g/g) Lösung von Silikon-Polymeren in Heptan (entspricht 9 g Bio-PSA 7-4201 und 9 g BIO-PSA 7-4301) gegeben. Die Mischung wurde zur Herstellung einer homogenen Dispersion mit einem Ultraturrax bei 10 000 UpM für 1 Minute gerührt. Anschließend wurde die Rotigotin-haltige Silikonmasse auf eine Scotch Pak 1109 Folie ausgestrichen (6 mm/Sec) und für 30 Minuten bei 50°C getrocknet. Schließlich wurde Schutzfolie (MN 19) aufgetragen.

### 4. Beispiel: Bestimmung des Wirkstoffflusses im Mäusehautmodell

Für die Fluxmessungen durch Mäusehaut wurde Bauch und Rückenhaut einer Dicke von ca. 120 bis 150 µm verwendet. Ein TTS mit einer ausgestanzten Fläche von 2,55 cm² wird in einer horizontalen Diffusionszelle auf die Hornschichtseite der Bauch- und Rückenhaut haarloser Mäuse fixiert. Unmittelbar anschließend wird die Akzeptorkammer der Zelle mit auf 32°C vortemperierter Phosphat-Pufferlösung (0,066 molar), pH 6,2, luftblasenfrei befüllt und das Freisetzungsmedium auf 32 ± 0,5°C thermostatisiert. Zu den Probeentnahmezeiten wird das Freisetzungsmedium gegen frisches, auf 32 ± 0,5°C thermostatisiertes Medium ausgetauscht. Die Rotigotine-Freisetzung wird per HPLC bestimmt.

### 5. Beispiel: Bestimmung des Wirkstoffflusses im Humanhautmodell

Die Bestimmung des Rotigotinfluxes durch Humanhaut wurde im wesentlichen durchgeführt wie in H. Tanojo et al, J. Control Rel. 45 (1997) 41-47 beschrieben.

Hierzu wurde Humanhaut in einer Dicke von 250 µm aus dem Abdomen gewonnen. Ein TTS mit einer Fläche von 2,545 cm² wurde auf Humanhaut gleicher Fläche aufgebracht, wobei die Haut zur Akzeptorseite hin auf einer Silikonmembran aufliegt. Als Akzeptorphase wurde PBS (0,066 molar) bei PH 6,2 und einer Temperatur von 32±0.5°C verwendet. Die Experimente wurden mit einem Flux von 5mL/h über 72 Stunden durchgeführt. Zu den Probenentnahmezeiten wird das Freisetzungsmedium gegen frisches, auf 32±0.5°C thermostatiertes Medium ausgetauscht und die Menge des freigesetzten Rotigotins per HPLC gemessen. Die Bestimmung der Fluxrate Q(t) erfolgte bezogen auf die Fläche der Meßzelle (0.552 cm²).

## Patentansprüche

1. Matrix zur transdermalen Verabreichung von Rotigotin [(-)-5,6,7,8-Tetrahydro-6-[propyl[2-(2-thienyl)ethyl]amino]-1-naphtol], enthaltend ein mit Rotigotin-Base übersättigtes Matrixpolymer, **dadurch gekennzeichnet, dass** der nicht im Matrixpolymer gelöste Anteil des Rotigotins als amorphe Partikel mit einem mittleren Durchmesser von maximal 30 µm im Matrixpolymer dispergiert ist und die Matrix frei von Löslichkeitsvermittlern, Kristallisationsinhibitoren und Dispersionsmitteln ist, mit der Maßgabe, dass die Matrix keinen heißschmelzfähigen Haftkleber enthält.

2. Matrix zur transdermalen Verabreichung von Rotigotin [(-)-5,6,7,8-Tetrahydro-6-[propyl[2-(2-thienyl)ethyl]amino]-1-naphtol], bestehend aus
(a) Matrixpolymer,
(b) Rotigotin-Base in einer Konzentration oberhalb der Löslichkeitsgrenze des Matrixpolymers, wobei der nicht im Matrixpolymer gelöste Anteil des Rotigotins als amorphe Partikel mit einem mittleren Durchmesser von maximal 30 µm im Matrixpolymer dispergiert ist und
(c) optional einem oder mehreren Antioxidanzien, mit der Maßgabe, dass die Matrix keinen heißschrnelzfähigen Haftkleber enthält.

3. Matrix nach einem der vorhergehenden Ansprüche, wobei das Matrixpolymer ein aminoresistentes Silikon oder eine Mischung aminoresistenter Silikone ist.

4. Matrix nach einem der vorhergehenden Ansprüche, **dadurch gekennzeichnet, dass** die Matrix selbstklebend ist.

5. Matrix nach einem der vorhergehenden Ansprüche **dadurch gekennzeichnet, dass** die Matrix besteht aus
(a) 60-95 Gew% eines aminoresistenten Silikons oder einer aminoresistenten Silikonmischung,
(b) 5-40 Gew% im Silikon dispergierte amorphe Rotigotin-Base und
(c) 0-2 Gew% Antioxidans.

6. Flächenförmige Vorrichtung zur transdermalen Verabreichung von Rotigotin, enthaltend eine Matrix nach einem der vorhergehenden Ansprüche sowie eine Rotigotin-undurchlässige Rückschicht.

7. Flächenförmige Vorrichtung nach Anspruch 5, **dadurch gekennzeichnet, dass** der Beladungsgrad mit Rotigotin zwischen 0.3 und 6 mg/cm² liegt.

8. Verwendung einer Vorrichtung oder einer Matrix nach einem der vorhergehenden Ansprüche zur Herstellung eines Arzneimittels zur Behandlung von Morbus Parkinson oder dem Restless Leg Syndrom.

9. Verwendung einer Vorrichtung oder einer Matrix nach einem der vorhergehenden Ansprüche zur Herstellung eines Arzneimittels zur Behandlung von Depressionen.

10. Verfahren zur Herstellung einer pharmazeutischen Matrix zur transdermalen Verabreichung von Rotigotin, **gekennzeichnet durch** die aufeinanderfolgenden Schritte:
(a) Auflösen von Matrixpolymer in einem Lösemittel,
(b) Zugabe von Rotigotin-Base in kristalliner Form in einer Menge oberhalb der Löslichkeitsgrenze des in (a) verwendeten Matrixpolymers,
(c) Entfernen des Lösemittels und Erwärmen der hergestellten Matrixmasse auf eine Temperatur von mindestens 74°C, bis das Rotigotin geschmolzen ist,
(d) Abkühlen der Matrixmasse, mit der Maßgabe, dass die Matrix keinen heißschmelzfähigen Haftkleber enthält.

11. Verfahren nach Anspruch 10, wobei die in Schritt (b) entstehende, mit Rotigotin übersättigte Polymermasse auf einer Rotigotin-undurchlässigen Folie ausgestrichen wird und sodann, wie in den Schritten (c) und (d) des Anspruchs 10 beschrieben, weiterbehandelt wird.

12. Verfahren nach einem der vorhergehenden Ansprüche, **dadurch gekennzeichnet, dass** das Matrixpolymer eine Löslichkeit für Rotigotin von < 3 Gew% hat.

13. Verfahren nach einem der vorhergehenden Ansprüche, **dadurch gekennzeichnet, dass** das Matrixpolymer ein aminoresistentes Silikon ist.

14. Verfahren nach einem der vorhergehenden Ansprüche, **dadurch gekennzeichnet, dass** das Matrixpolymer ein aminoresistenter Silikonhaftkleber oder eine Mischung mehrerer aminoresistenter Silikonhaftkleber ist.

## Claims

1. Matrix for transdermal administering of rotigotine [(-)-5,6,7,8-tetrahydro-6-[propyl [2-(2-thienyl)ethyl]amino]-1-naphtol], containing a matrix polymer supersaturated with rotigotine base, **characterized in that** the portion of the rotigotine not dissolved in the matrix polymer is dispersed in the matrix polymer as amorphous particles with a maximum mean diameter of 30 µm and the matrix is free of solvents, crystallization inhibitors and dispergents, with the proviso, that the matrix does not contain a hotmelt adhesive.

2. Matrix for transdermal administering of rotigotine [(-)-5,6,7,8-tetrahydro-6-[propyl [2-(2-thienyl)ethyl] amino]-1-naphtol], consisting of
(a) matrix polymer,
(b) rotigotine base in a concentration above the solubility limit of the matrix polymer, wherein the portion of the rotigotine not dissolved in the matrix polymer is dispersed in the matrix polymer as amorphous particles with a maximum mean diameter of 30 µm and
(c) optionally one or more antioxidants,
with the proviso, that the matrix does not contain a hotmelt adhesive.

3. Matrix according to one of the preceding claims, wherein the matrix polymer is a amino-resistant silicon or a mixture of amino-resistant silicons.

4. Matrix according to one of the preceding claims, **characterized in that** the matrix is self-adhesive.

5. Matrix according to one of the preceding claims, **characterized in that** the matrix consists of
(a) 60-95 weight percent of an amino-resistant silicon or an amino-resistant silicon mixture,
(b) 5-40 weight percent amorphous rotigotine base dispersed in the silicon and
(c) 0-2 weight percent antioxidant.

6. Planiform system for transdermal administering of rotigotine, containing a matrix according to one of the preceding claims and a backing impermeable to rotigotine.

7. Planiform system according to claim 5, **characterized in that** the rotigotine charge is between 0.3 and 6 mg/cm².

8. Use of a system or a matrix according to one of the preceding claims to produce a drug to treat Morbus Parkinson or Restless Leg Syndrome.

9. Use of a system or a matrix according to one of the preceding claims to produce a drug to treat depression.

10. Method for producing a pharmaceutical matrix for transdermal administering of rotigotine, **characterized by** the consecutive steps:
(a) dissolving matrix polymer in a solvent,
(b) adding rotigotine base in crystalline form in a quantity above the solubility limit of the matrix polymer used in (a),
(c) removing the solvent and heating the matrix mass produced to a temperature of at least 74°C until the rotigotine has melted,
(d) cooling the matrix mass,
with the proviso, that the matrix does not contain a hotmelt adhesive.

11. Method according to claim 10, wherein the polymer mass supersaturated with rotigotine - created in step (b) - is applied on a foil impermeable to rotigotine and then, as described in steps (c) and (d) of claim 10, is further treated.

12. Method according to one of the preceding claims, **characterized in that** the matrix polymer has a solubility for rotigotine of <3 weight percent.

13. Method according to one of the preceding claims, **characterized in that** the matrix polymer is an amino-resistant silicon.

14. Method according to one of the preceding claims, **characterized in that** the matrix polymer is an amino-resistant silicon contact adhesive or a mixture of several amino-resistant silicon contact adhesives.

## Revendications

1. Matrice pour l'administration transdermique de rotigotine [(-)-5,6,7,8-tétrahydro-6-[propyl[2-(2-thiényl)éthyl]amino]-1-naphtol], contenant un polymère de matrice sursaturé de rotigotine base, ***caractérisée en ce que*** la fraction de la rotigotine non dissoute dans le polymère de matrice est dispersée, sous la forme de particules amorphes ayant un diamètre moyen de 30 µm au maximum, dans le polymère de matrice, et la matrice ne contient pas d'activateurs de dissolution, d'inhibiteurs de cristallisation ni d'agents de dispersion, à la condition que la matrice ne contienne pas d'adhésifs thermofusibles.

2. Matrice pour l'administration transdermique de rotigotine [(-)-5,6,7,8-tétrahydro-6-[propyl [2-(2-thiényl)éthyl]amino]-1-naphtol], composée de :
(a) polymère de matrice,
(b) rotigotine base à une concentration supérieure à la limite de solubilité du polymère de matrice, la fraction de la rotigotine non dissoute dans le polymère de matrice étant dispersée, sous forme de particules amorphes ayant un diamètre moyen de 30 µm au maximum, dans le polymère de matrice, et
(c) facultativement, un ou plusieurs antioxydants,
à la condition que la matrice ne contienne aucun adhésif thermofusible.

3. Matrice selon l'une quelconque des revendications précédentes, dans laquelle le polymère de matrice est un silicone résistant aux amines ou un mélange de silicones résistants aux amines.

4. Matrice selon l'une quelconque des revendications précédentes, ***caractérisée en ce que*** la matrice est autocollante.

5. Matrice selon l'une quelconque des revendications précédentes, ***caractérisée en ce que*** la matrice se compose de :
(a) 60 à 95 % en poids d'un silicone résistant aux amines ou d'un mélange de silicones résistants aux amines,
(b) 5 à 40 % en poids de rotigotine base amorphe en dispersion et
(c) 0 à 2 % en poids d'antioxydant.

6. Dispositif plan pour l'administration transdermique de rotigotine, comportant une matrice selon l'une quelconque des revendications précédentes et une couche de support imperméable à la rotigotine.

7. Dispositif plan selon la revendication 5, ***caractérisé en ce que*** la charge de rotigotine est comprise entre 0,3 et 6 mg/cm².

8. Utilisation d'un dispositif ou d'une matrice selon l'une quelconque des revendications précédentes pour la fabrication d'un médicament destiné au traitement de la maladie de Parkinson ou du syndrome des jambes sans repos.

9. Utilisation d'un dispositif ou d'une matrice selon l'une quelconque des revendications précédentes pour la fabrication d'un médicament destiné au traitement des dépressions.

10. Procédé de fabrication d'une matrice pharmaceutique pour l'administration transdermique de rotigotine, ***caractérisé en ce qu'**il* comprend les étapes suivantes :
(a) dissolution de polymère de matrice dans un solvant,
(b) ajout de rotigotine base sous forme cristalline dans une quantité dépassant la limite de solubilité du polymère de matrice utilisé à l'étape (a),
(c) élimination du solvant et chauffage de la pâte de matrice obtenue à une température d'au moins 74°C, jusqu'à la fusion de la rotigotine,
(d) refroidissement de la pâte de matrice,
à la condition que la matrice ne contienne aucun adhésif thermofusible.

11. Procédé selon la revendication 10, dans lequel la pâte de polymère sursaturée de rotigotine obtenue à l'étape (b) est appliquée sur un film imperméable à la rotigotine, puis traitée de la manière décrite aux étapes (c) et (d) de la revendication 10.

12. Procédé selon l'une quelconque des revendications précédentes, ***caractérisé en ce que*** le polymère de matrice présente une solubilité pour la rotigotine inférieure à 3 % en poids.

13. Procédé selon l'une quelconque des revendications précédentes, ***caractérisé en ce que*** le polymère de matrice est un silicone résistant aux amines.

14. Procédé selon l'une quelconque des revendications précédentes, ***caractérisé en ce que*** le polymère de matrice est un adhésif au silicone résistant aux amines ou un mélange de plusieurs adhésifs au silicone résistants aux amines.
